# EUROPEAN PATENT APPLICATION

(11) **EP 4 755 434 A1**
(43) Date of publication of application: **10.06.2026**
(21) Application number: 24848172.3
(22) Date of filing: 26.07.2024
(51) Int. Cl.: A61M 25/088, A61B 17/22

(54) **NAVIGATION-ASSISTED THROMBUS RETRIEVAL SYSTEM USED IN CEREBRAL VESSEL**

(30) Priority: 01.08.2023 CN 202310954037
(71) Applicant: Infinity Neuro China Co., Ltd, Suzhou, Jiangsu 215127 (CN)
(72) Inventor: LIU, Yichen, Suzhou, Jiangsu 215127 (CN); FERRERA, David, Suzhou, Jiangsu 215127 (CN)
(74) Representative: ABG Intellectual Property Law, S.L.
(86) International application number: PCT/CN2024/107798
(87) International publication number: WO 2025/026215

(57) **Abstract**

A navigation-assisted thrombus retrieval system used in a cerebral vessel, comprising a reperfusion device and a navigation device. The navigation device is used for guiding the reperfusion device to smoothly move in the cerebral vessel; the distal end of the navigation device passes through the proximal end of the reperfusion device and reaches the distal end of the reperfusion device; and the outer diameter of the navigation device fits with the inner diameter of the reperfusion device, so that the reperfusion device axially extends along the axis of the navigation device and moves in the cerebral vessel along with the navigation device. The navigation device is used for navigating the reperfusion device to move in the cerebral vessel, the distal end of the navigation device is a wedge-shaped tip, and the flexible and freely-bending wedge-shaped tip can turn instantly when passing through an ophthalmic artery bend, such that the reperfusion device can easily pass through the cerebral vessel; and the outer diameter of the navigation device fits with the inner diameter of the reperfusion device, such that the reperfusion device and the navigation device are always aligned in the same axial direction when moving in the cerebral vessel, and the reperfusion device smoothly passes through the ophthalmic artery bend under the action of the navigation device and does not cause damage to the wall of the vessel.

## Description

The present application claims priority to the Chinese Patent Application No. 202310954037.7, titled "NAVIGATION-ASSISTED THROMBUS RETRIEVAL SYSTEM USED IN CEREBRAL VESSEL", filed on August 1, 2023 with the China National Intellectual Property Administration, which is incorporated herein by reference in its entirety.

### FIELD

The present application relates to the technical field of medical instruments, and in particular to a navigation-assisted thrombectomy system for use in a cerebral vessel.

### BACKGROUND

Cerebral thrombosis may occur in any segment of the cerebral vessels, and in clinical practice, it is particularly common at bifurcations of the internal carotid artery, the anterior cerebral artery, and the middle cerebral artery. The internal carotid artery is connected to three main arteries, namely the middle artery, the anterior artery, and the posterior communicating artery and the posterior artery. The cerebral thrombosis occurs most frequently at a terminal of the internal carotid artery and in the middle cerebral artery, which accounts for over 65% of acute ischemic stroke cases. A smooth advancement of a large-diameter aspiration catheter through the vessel segment close to the ophthalmic artery to the site of occlusion is crucial for vascular recanalization. A challenge for an aspiration catheter during thrombus aspiration lies in that, when the catheter is advanced in the blood vessel, the overall hardness of the catheter increases after continuous turning, making it difficult to further turn a tip of the catheter. Moreover, the tip of the catheter is a circular ring. When an internal guidewire fails to turn the catheter sufficiently, a side of the circular ring may catch on a bifurcation of the blood vessel, which is known as the "ledge effect". As a result, the obstructed catheter cannot pass by the bifurcation even under an additional pushing force. If an external force is forcibly applied, a middle section of the catheter may be bent significantly in the blood vessel, causing a risk of damaging a vascular wall and thus resulting in vascular dissection and subarachnoid hemorrhage. In China, the majority of patients with cerebral thrombosis are the elderly, who have poor vascular conditions and often suffer from vascular stenosis or arterial plaques. Therefore, when the catheter passes by the vascular wall, it is likely to result in plaque detachment, which poses a risk of secondary stroke.

Currently, there are two aspiration catheter products on the market for addressing the above issues. One is Penumbra's Jet 7, the tip of which can be bent in the case where the catheter is obstructed during movement, allowing it to smoothly pass through a vessel bend close to the ophthalmic artery. The other one is Sofia, the tip of which can also be bent to pass through the bend close to the ophthalmic artery. Both Penumbra's Jet 7 and Sofia have an angiography ring at the catheter tip, which is made of hard metal. Hence, there is a risk of vascular wall detachment when the tip moves in the blood vessel along the vascular wall. Moreover, an additional pushing force is required to assist the catheter tip in turning and passing through the bend close to the ophthalmic artery, which inevitably causes damage to the artery at the bifurcations.

The patent document CN113081166A discloses a micro catheter used to guide a reperfusion device to pass through the tortuous cerebral vessel, particularly pass by the bifurcation point at the origin of the ophthalmic artery. The micro catheter includes a body and a protrusion. The body is inserted in the reperfusion device, and an angiography part is provided on the body to monitor the position of the body and the protrusion in real time. The protrusion is sleeved on the body, and an outer wall of the protrusion is in clearance fit with an inner wall of the reperfusion device. An axis of the reperfusion device coincides with an axis of the body, preventing radial movement of the reperfusion device, thereby ensuring the coaxiality of the cerebral vessel, the body, and the reperfusion device to prevent the catheter wall of the reperfusion device from scratching the cerebral vessel. When the reperfusion device follows the pathway established by the micro catheter and moves to the narrow and tortuous bifurcation of the cerebral vessel, the reperfusion device and the cross section of the micro catheter are concentric, preventing an end wall of the reperfusion device from catching on the bifurcation of the cerebral vessel. Although this patent has achieved effects, the angiography ring thereof is also provided at the tip of the catheter and is made of metal, which is the same as the existing product, and thus the risk of damaging the inner wall of the blood vessel still exists.

Therefore, it is needed to address the above technical issues, so that the reperfusion device can be quickly and smoothly advanced to the occlusion site, the damage to the inner wall of the blood vessel can be avoided, and the thrombectomy efficiency can be improved.

### SUMMARY

To address the above technical issues in the related art, i.e., how to advance the reperfusion device quickly and smoothly through the blood vessel to the location of a thrombus while preventing the catheter from damaging the vascular wall, a navigation-assisted thrombectomy system for use in a cerebral vessel is provided in the present application, including a reperfusion device and a navigation device.

A main body of the reperfusion device is a catheter. The reperfusion device includes an inner liner, a braided layer, and an outer coating. The reperfusion device is omnidirectionally bendable to 360 degrees without fracture.

A distal end of the navigation device is configured to be inserted from a proximal end of the reperfusion device to a distal end of the reperfusion device.

An outer radial side of the navigation device is in fit with an inner radial side of the reperfusion device. The reperfusion device extends along an axis of the navigation device and is configured to move along with the navigation device in the cerebral vessel.

The navigation device has a cylindrical main body. The distal end of the navigation device is configured as a wedge-shaped tip. The wedge-shaped tip is configured to guide the reperfusion device to bend omnidirectionally to pass by an ophthalmic artery and move to a location of a thrombus. The wedge-shaped tip has a length of 1 to 5 cm.

The wedge-shaped tip is visible under angiography for positioning of the thrombus.

The wedge-shaped tip of the navigation device is configured to assist in pulling the thrombus back into the reperfusion device through friction between the wedge-shaped tip and the thrombus, to mechanically assist the reperfusion device in aspirating the thrombus.

The wedge-shaped tip is made of a flexible material, and a length of the wedge-shaped tip is preferably 2 to 3.5 cm.

The wedge-shaped tip is made of a polymer composite material and is integrally formed by thermal processing. The wedge-shaped tip is omnidirectionally bendable to 360 degrees without fracture.

The reperfusion device consists of 10 to 60 segments and has a hardness that gradually decreases from the proximal end of the reperfusion device to the distal end of the reperfusion device. A rate of change in hardness between two adjacent segments arranged from the distal end of the reperfusion device to the proximal end of the reperfusion device is less than 75%, preferably less than 67%.

A main body of the reperfusion device is a catheter, and the catheter has an outer diameter ranging from 0.8 to 0.12 inches, preferably from 0.85 to 0.115 inches.

The catheter, which serves as the main body of the reperfusion device, has a length ranging from 110 to 150 cm, preferably from 120 to 145 cm.

In an embodiment, the outer radial side of the navigation device is in fit with the inner radial side of the reperfusion device. A difference between an outer diameter of the navigation device and an inner diameter of the reperfusion device is smaller than or equal to 0.005 inches, so as to cooperate with the wedge-shaped tip of the navigation device to eliminate a ledge effect.

A polymer composite material of the wedge-shaped tip contains contrast agent particles.

In an embodiment, the navigation-assisted thrombectomy system further includes a distal access guide device. The distal access guide device is configured to be advanced to a distal-most vascular position before the navigation device enters the cerebral vessel.

The navigation device and the reperfusion device are configured to pass through the distal access guide device, pass through a vessel bend close to an ophthalmic artery, and enter the cerebral vessel.

A core layer of the distal access guide device consists of 5 to 40 segments. The distal access guide device has a hardness that gradually decreases from a proximal end of the distal access guide device to a distal end of the distal access guide device. A proximal-most segment of the distal access guide device has a maximum hardness, and a distal-most segment of the distal access guide device has a minimum hardness. A rate of change in hardness between two adjacent segments arranged from the distal end of the distal access guide device to the proximal end of the distal access guide device is less than 75%, preferably less than 67%.

In an embodiment, before the navigation device enters the cerebral vessel, the distal access guide device is configured to be advanced to the distal-most vascular position.

In an embodiment, the navigation-assisted thrombectomy system for use in a cerebral vessel further includes a control handle, which is configured for operation and control of a guiding catheter and a thrombus aspiration catheter.

The advantages and beneficial effects of the technical solutions of the present application are as follows.
(1) In the aspiration device for cerebral thrombi aspiration according to the present application, a navigation device is provided to guide the reperfusion device to move in the cerebral vessel. The distal end of the navigation device is a wedge-shaped tip, which is flexible and can be freely bent, and the wedge-shaped tip can make a turn instantly at the vessel bend close to the ophthalmic artery and thus easily pass through the vessel bend. The outer radial side of the navigation device is in fit with the inner radial side of the reperfusion device, ensuring that the reperfusion device and the navigation device always move along the same axis in the cerebral vessel. Under the action of the navigation device, the reperfusion device can smoothly pass through the vessel bend close to the ophthalmic artery, which avoids the "ledge effect" when the reperfusion device passes by the bifurcations of the cerebral vessel, such as a bifurcation where the ophthalmic artery originates, thereby effectively preventing the reperfusion device from compressing and damaging the vascular wall and improving the efficiency of the advancement of the reperfusion device into place.
(2) The reperfusion device is configured to always move through the central pathway of the cerebral vessel, and has a good flexibility, which is not likely to scratch the vascular wall, thereby avoiding the risk of plaque detachment caused by the catheter passing by the vascular wall, which would otherwise pose a risk of secondary stroke.
(3) The auxiliary catheter according to the present application has the wedge-shaped tip. Compared with existing products, the guide catheter according to the present application has no angiography ring. Therefore, under an external pushing force, the auxiliary catheter can easily pass through the complex vascular pathways in the cerebral vessel without scratching the vascular wall, avoiding the damage to the blood vessel. Further, the whole wedge-shaped tip is visible under angiography and thus has an indication function, which can help to clearly position the thrombus, so that the thrombus can be pulled by the wedge-shaped tip through the mechanical design of the wedge-shaped tip.
(4) According to the product described in the present application, with the quick navigation of the distal access guide device in combination with the flexible wedge-shaped tip, the surgical efficiency is greatly improved, and the thrombectomy process can be finished within 10 to 15 minutes, which is more than twice as fast as that in the related art.

The above is merely a brief description of the technical solutions of the present application. In order to enable better understanding of the technical means of the present application to enable implementations thereof according to the content of the specification, and in order to make the above and other purposes, features, and advantages of the present application more obvious and understandable, the preferred embodiments of the present application and the accompanying drawings are described in detail below.

Based on the detailed description of the specific embodiments of the present application in conjunction with the accompanying drawings, those skilled in the art will have a better understanding of the above and other purposes, advantages, and features of the present application.

### BRIEF DESCRIPTION OF THE DRAWINGS

In order to provide a clearer explanation of embodiments of the present application or technical solutions in the related art, a brief introduction of the accompanying drawings required for description of the embodiments or the related art will be given below. Obviously, the accompanying drawings in the following description only show some embodiments of the present application. For those skilled in the art, other drawings can be obtained based on these drawings without creative labor. Similar elements or parts throughout the drawings are generally identified by similar reference numerals. In the drawings, each element or part may not be drawn to scale.
FIG. 1 is a schematic structural view of a navigation-assisted thrombectomy system for use in a cerebral vessel according to the present application;
FIG 2 is a schematic view of a wedge-shaped tip of the navigation-assisted thrombectomy system for use in the cerebral vessel according to the present application;
FIG 3 is a schematic view showing a state of the navigation-assisted thrombectomy system in the cerebral vessel according to the present application;
FIG. 4 is a stiffness data chart of a reperfusion device according to the present application in comparison with a control group (Tenzig); and
FIG. 5 is a stiffness data chart of the reperfusion device in FIG. 4 at a distal end (0-50 cm) according to the present application in comparison with the control group.

Numeral references are listed below:

| | | | |
|---|---|---|---|
| 1 | navigation device, | 2 | reperfusion device, |
| 3 | distal access guide device, | 4 | control handle. |

### DETAILED DESCRIPTION OF THE EMBODIMENTS

In order to make the purpose, technical solutions, and advantages of the embodiments of the present application more clear, the technical solution in the embodiments of the present application are described clearly and completely described in conjunction with the accompanying drawings. Obviously, the described embodiments are only part of the embodiments of the present application, not all of them. In the following description, specific configurations and details of components are merely provided to assist in a comprehensive understanding of the embodiments of the present application. Therefore, those skilled in the art should understand that various changes and modifications can be made to the embodiments described herein without departing from the scope and spirit of the present application. In addition, for clarity and conciseness, descriptions of known functions and constructions have been omitted in the embodiments.

It should be understood that the term "one embodiment" or "the present embodiment" mentioned throughout the specification means that specific features, structures, or characteristics related to the embodiments are included in at least one embodiment of the present application. Therefore, the uses of "one embodiment" or "this embodiment" throughout the entire specification do not necessarily refer to the same embodiment. In addition, these specific features, structures, or characteristics may be combined in any suitable manner in one or more embodiments.

In addition, the present application may repeatedly refer to numbers and/or letters in different examples. Such repetitions are for the purpose of simplification and clarity, which does not indicate the relationship between the various embodiments and/or configurations discussed.

The term "and/or" herein is only a description of the association relationship between related objects, indicating that there can be three types of relationships. For example, A and/or B can represent: the existence of A alone, the existence of B alone, and the existence of both A and B. The term "/and" in this article describes another type of association relationship, indicating that there can be two types of relationships. For example, A /and B can represent: the existence of A alone, and the existence of both A and B. In addition, the character "/" herein generally indicates that the related former and latter objects are in an "or" relationship.

The term "at least one" used herein is merely a description of the association relationship between related objects, indicating that there can be three types of relationships. For example, at least one of A and B can represent three cases, namely the existence of A alone, the existence of both A and B, and the existence of B alone.

It should also be noted that relational terms such as first and second are merely used herein to distinguish one entity or operation from another, and do not necessarily require or imply any actual relationship or order between these entities or operations. Moreover, the terms "including", "containing", or any other variation thereof are intended to encompass non-exclusive inclusion.

### First Embodiment

A navigation-assisted thrombectomy system for use in a cerebral vessel is provided in this embodiment.

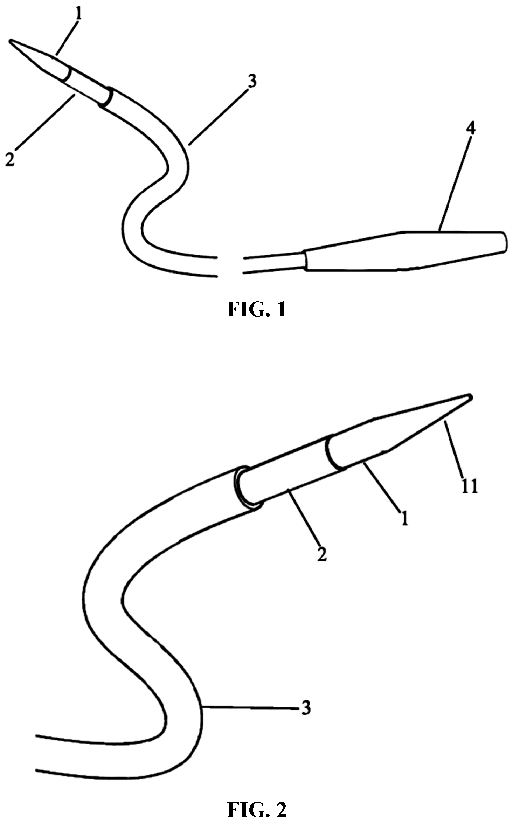

As shown in FIGS. 1, 2, and 3, a navigation-assisted thrombectomy system for use in a cerebral vessel is provided, which includes a navigation device 1 and a reperfusion device 2. The navigation device 1 is configured to guide the reperfusion device 2 smoothly through the cerebral vessel.

A distal end of the navigation device 1 is configured to be inserted from a proximal end of the reperfusion device 2 to a distal end of the reperfusion device 2.

An outer radial side of the navigation device 1 is in fit with an inner radial side of the reperfusion device 2, and the reperfusion device 2 extends along an axis of the navigation device 1 and is configured to move along with the navigation device 1 in the cerebral vessel.

The reperfusion device includes an inner liner, a braided layer, and an outer coating. The reperfusion device is omnidirectionally bendable to 360 degrees without fracture.

The navigation device 1 has a cylindrical main body. The distal end of the navigation device 1 is configured as a wedge-shaped tip 11. The wedge-shaped tip 11 is configured to guide the reperfusion device 2 to bend omnidirectionally, to pass by the ophthalmic artery and move to a location of a thrombus. The wedge-shaped tip 11 has a length of 1 to 5 cm.

The whole wedge-shaped tip 11 is visible under angiography, which can be used to position the thrombus.

The wedge-shaped tip 11 of the navigation device 1 is configured to assist in pulling the thrombus back into the reperfusion device through friction between the wedge-shaped tip 11 and the thrombus, to mechanically assist the reperfusion device in aspirating the thrombus.

The wedge-shaped tip 11 is made of a flexible material, and the length of the wedge-shaped tip 11 is preferably 2 to 3.5 cm.

The wedge-shaped tip 11 is made of a polymer composite material and is integrally formed by thermal processing. The wedge-shaped tip 11 can be freely bent to 360 degrees without fracture.

The reperfusion device 2 consists of 10 to 60 segments. A hardness of the reperfusion device 2 gradually decreases from the proximal end to the distal end of the reperfusion device 2. A rate of change in hardness between two adjacent segments arranged from the distal end to the proximal end of the reperfusion device 2 is less than 75%, preferably less than 67%.

A main body of the reperfusion device 2 is a catheter. An outer diameter of the catheter ranges from 0.8 to 0.12 inches, preferably from 0.85 to 0.115 inches.

The catheter, as the main body of the reperfusion device 2, has a length of 110 to 150 cm, preferably 120 to 145 cm.

In an embodiment, the outer radial side of the navigation device 3 is in fit with the inner radial side of the reperfusion device 2, and a difference between an outer diameter of the navigation device 3 and an inner diameter of the reperfusion device 2 is smaller than or equal to 0.005 inches. The ledge effect can be eliminated through the cooperation of this configuration and the wedge-shaped tip of the navigation device.

The polymer composite material of the wedge-shaped tip 11 contains contrast agent particles.

In an embodiment, the navigation-assisted thrombectomy system further includes a distal access guide device 3. Before the navigation device enters the cerebral vessel, the distal access guide device is configured to be delivered to a distal-most vascular position.

The navigation device and the reperfusion device are configured to pass through the distal access guide device 3 and through a vessel bend close to an ophthalmic artery, and enter the cerebral vessel.

A core layer of the distal access guide device 3 consists of 5 to 40 segments. A hardness of the distal access guide device 3 gradually decreases from a proximal end to a distal end of the distal access guide device 3, with a hardness of a proximal-most segment of the distal access guide device 3 being the highest and a hardness of a distal-most segment of the distal access guide device 3being the lowest. A rate of change in hardness between two adjacent segments arranged from the distal end to the proximal end of the distal access guide device 3 is less than 75%, preferably less than 67%.

In an embodiment, before the navigation device enters the cerebral vessel, the distal access guide device 3 is configured to be delivered to a distal-most vascular position.

In an embodiment, a navigation-assisted thrombectomy system for use in a cerebral vessel further includes a control handle 4, which is configured for operation and control of a guide catheter and a thrombus-aspiration catheter.

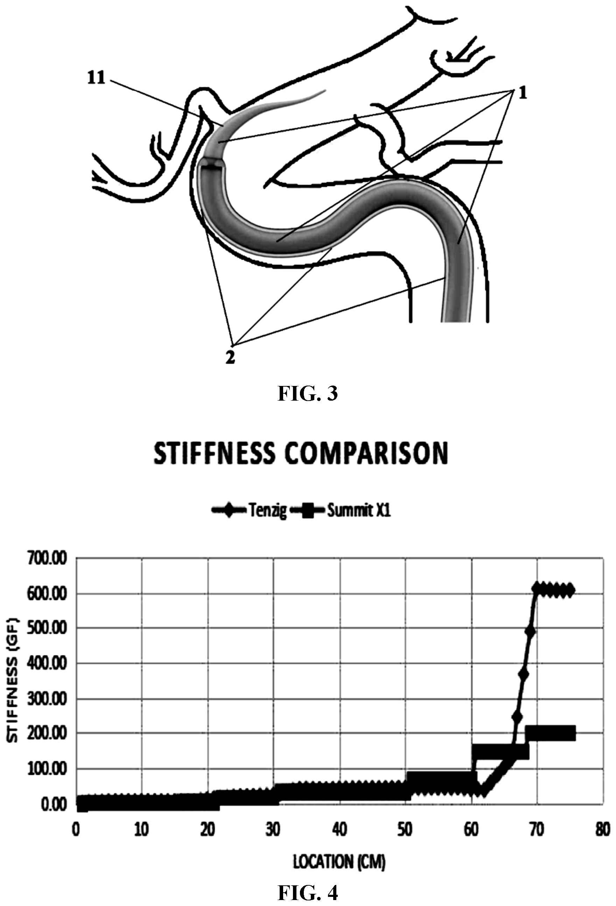

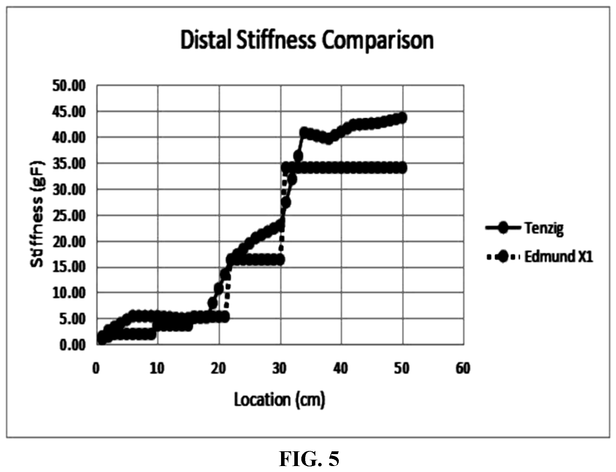

FIG. 4 is a stiffness data chart of the reperfusion device according to the present application in comparison with a control group (Tenzig), and FIG. 5 is a stiffness data chart of the reperfusion device at a distal end (0 to 50 cm) in FIG. 4 according to the present application in comparison with the control group. As can be seen from FIG. 4, the reperfusion device in the present application is more flexible as a whole. A stiffness of the reperfusion device according to the present application at the proximal end is lower than 250gF, while a stiffness of the reperfusion device of the control group is higher than 500gF. As can be seen from FIG. 5, the distal end of the reperfusion device according to the present application is more flexible. The stiffness of the reperfusion device according to the present application at the proximal end is lower than 250gF, while the stiffness of the reperfusion device of the control group is higher than 500gF. Within the range of 0 to 10 cm at the distal end, the stiffness of the reperfusion device according to the present application is also much lower than that of the control group.

### Second Embodiment

As shown in FIGS. 1 and 2, the navigation-assisted thrombectomy system further includes a distal access guide device 3. Before the navigation device 1 enters the cerebral vessel, the distal access guide device 3 is configured to be delivered to and stopped at a distal-most position in the blood vessel.

Before the navigation device 1 is advanced into the blood vessel, the distal access guide device 3 is first delivered into the blood vessel and moved to a predetermined position. Then, the navigation device 1 and the reperfusion device 2 pass through the distal access guide device 3 to move into the blood vessel and then into the cerebral vessel.

The distal end of the navigation device 1 is configured as the wedge-shaped tip 11. The wedge-shaped tip 11 is made of a polymer composite material and is integrally formed by thermal processing. The polymer composite material of the wedge-shaped tip 11 contains contrast agent particles.

Further, the wedge-shaped tip 11 of the navigation device 1 is made of a flexible material, which has a good flexibility and can be freely bent. When passing by the ophthalmic artery, the wedge-shaped tip can generate an elastic force under stress, thereby turning instantly. Accordingly, the reperfusion device follows the navigation device and also smoothly passes by the ophthalmic artery to reach the location of the thrombus.

The wedge-shaped tip 11 is formed by thermal processing. The distal end of the guide catheter 1 is configured as a wedge-shaped tip, and the proximal end of the guide catheter 1 is of a hollow catheter structure. The variations in stiffness and tensile stiffness of the navigation device 1 from the wedge-shaped tip at the distal end to the proximal end are shown in FIG. 4. As can be seen from the figure, the navigation device has good stiffness and tensile stiffness performance.

Further, the wedge-shaped tip 11 is integrally formed by thermal processing. The contrast agent is added to the raw material before the formation of the wedge-shaped tip 11. After the wedge-shaped tip 11 is formed by processing, the angiography ring is embedded in the wedge-shaped tip 11. Moreover, the contrast agent may be distributed throughout the wedge-shaped tip 11 or distributed at a leading end and a tail end of the wedge-shaped tip 11, which facilitates real-time monitoring of the position of the navigation device in the cerebral vessel.

Due to the processing method of the wedge-shaped tip and the configuration of the contrast agent above, when the reperfusion device is guided by the guide catheter according to the present application to move in the cerebral vessel, it will not cause the issue occurring in existing products, where the hard metal angiography ring on the catheter hinders the catheter movement in the cerebral vessel, especially at bifurcations, risking abrasion of the inner vascular wall.

In this embodiment, an outer diameter of the reperfusion device 2 is smaller than an inner diameter of the cerebral vessel. During surgery, the navigation device 1 is first controlled to move a distance in the cerebral vessel, and the position of the navigation device is monitored in real time with the contrast agent, so as to ensure that an axis of the navigation device 1 coincides with an axis of the cerebral vessel. This prevents a catheter wall of the reperfusion device 2 from scratching a cerebral vascular wall caused by the offset of the navigation device 1 during subsequent surgery, or prevents an end wall of the reperfusion device 2 from catching on the bifurcation of the cerebral vessel caused by the offset of the navigation device during subsequent surgery, which would otherwise blocks the reperfusion device 2 from moving.

In this embodiment, the distal end of the guide catheter is configured as the wedge-shaped tip, and the outer radial side of the navigation device is in fit with the inner radial side of the reperfusion device to form an interference fit. In this way, the reperfusion device can be kept in axial alignment with the navigation device under the action of the navigation device. The flexible and omnidirectionally bendable wedge-shaped tip can make a turn instantly at the vessel bend close to the ophthalmic artery and thus easily pass through the vessel bend. Moreover, under the guidance of the navigation device, it can prevent the "ledge effect" when the reperfusion device passes by the bifurcation of the cerebral vessel, such as the bifurcation where the ophthalmic artery originates, thereby avoiding compression and damage to the vascular wall and other risks.

### Third Embodiment

Based on the above embodiments, another structure of the navigation-assisted thrombectomy system for use in a cerebral vessel is provided in this embodiment.

As shown in FIG. 1, FIG. 1 is a schematic structural view of a navigation-assisted thrombectomy system for use in a cerebral vessel according to the present application.

A navigation-assisted thrombectomy system for use in a cerebral vessel includes a navigation device 1, a reperfusion device 2, a distal access guide device 3, and a control handle 4.

In this embodiment, the distal access guide device 1 is first advanced into the blood vessel and stops at a distal-most position in the vessel. The distal access guide device 1 serves as a support for the blood vessel to facilitate the subsequent operation of the reperfusion device. In this embodiment, the distal access guide device is a catheter with a large inner diameter. The catheter has a thin wall and a large diameter, which can provide maximum support to the blood vessel.

Further, a distal end of the navigation device 1 is inserted from a proximal end of reperfusion device 2 to a distal end of reperfusion device 2. An outer radial side of the navigation device 1 is in fit with an inner radial side of the reperfusion device 2, so that the reperfusion device 2 extends along an axis of the navigation device 1 and moves along with the navigation device 1 in the cerebral vessel.

The distal end of the navigation device 1 is configured as a wedge-shaped tip 11, and a contrast agent is provided in the wedge-shaped tip 11.

Further, the control handle 4 can be used to operate and control the reperfusion device and the navigation device.

### Fourth Embodiment

Based on the first and second embodiments, a method for using the navigation-assisted thrombectomy system for use in a cerebral vessel according to the present application is briefly described in this embodiment. The method includes the following steps.

In the preferred embodiment, the method for using the navigation-assisted thrombectomy system for use in the cerebral vessel according to the present application includes the following steps.

In Step 1, the distal access guide device is advanced into a blood vessel to reach a distal-most position in the vessel, and the navigation device and the reperfusion device are advanced into the blood vessel along the distal access guide device and then into the cerebral vessel;

In Step 2, the navigation device is moved in the cerebral vessel, the reperfusion device is guided by the navigation device to pass by the bifurcations of the cerebral vessel along the same axis as the navigation device to reach the location of the thrombus in the cerebral vessel. At this time, the wedge-shaped tip of the navigation device penetrates into the thrombus, brings the thrombus into the reperfusion device under a withdrawal force of the control handle, and cooperates with a negative pressure aspiration device to aspirate the thrombus out of body.

In Step 3, after aspiration of the thrombus, the reperfusion device and the navigation device are withdrawn from the body by manipulation of the control handle.

By the structural product design provided in the present application, the flexible and omnidirectionally bendable wedge-shaped tip can make a turn instantly at the vessel bend close to ophthalmic artery and thus easily pass through it. Moreover, the outer radial side of the navigation device is in fit with the inner radial side of the reperfusion device, and thus the reperfusion device and the navigation device can always move coaxially in the cerebral vessel. Under the action of the navigation device, the reperfusion device also smoothly passes through the vessel bend close to the ophthalmic artery, which prevents the "ledge effect" when the reperfusion device passes by the of the cerebral vessel, such as a bifurcation where the ophthalmic artery originates. Therefore, the reperfusion device can move to the location where the vascular blockage occurs more quickly and move smoothly in the cerebral vessel while not causing less damage to the blood vessel.

It should be understood that the above specific embodiments of the present application are merely for illustrative purposes or to explain the principles of the present application, and do not constitute limitations on the present application. Therefore, any modifications, equivalent substitutions, improvements, etc. made without departing from the spirit and scope of the present application shall be included within the protection scope of the present application. In addition, the appended claims of the present application are intended to cover all variations and modifications falling within the scope and boundary of the attached claims, or equivalent forms of such scope and boundary.

## Claims

1. A navigation-assisted thrombectomy system for use in a cerebral vessel, comprising:
a reperfusion device; and
a navigation device, wherein
a main body of the reperfusion device is a catheter, the reperfusion device comprises an inner liner, a braided layer, and an outer coating, and the reperfusion device is omnidirectionally bendable to 360 degrees without fracture;
a distal end of the navigation device is configured to be inserted from a proximal end of the reperfusion device to a distal end of the reperfusion device;
an outer radial side of the navigation device is in fit with an inner radial side of the reperfusion device, and the reperfusion device extends along an axis of the navigation device and is configured to move along with the navigation device in the cerebral vessel;
the navigation device has a cylindrical main body, the distal end of the navigation device is configured as a wedge-shaped tip, the wedge-shaped tip is configured to guide the reperfusion device to bend omnidirectionally to pass by an ophthalmic artery and move to a location of a thrombus, and the wedge-shaped tip has a length of 1 to 5 cm;
the wedge-shaped tip is visible under angiography for positioning of the thrombus; and
the wedge-shaped tip of the navigation device is configured to assist in pulling the thrombus back into the reperfusion device through friction between the wedge-shaped tip and the thrombus, to mechanically assist the reperfusion device in aspirating the thrombus.

2. The navigation-assisted thrombectomy system for use in the cerebral vessel according to claim 1, wherein
the wedge-shaped tip is made of a flexible material, and the length of the wedge-shaped tip is preferably 2 to 3.5 cm.

3. The navigation-assisted thrombectomy system for use in the cerebral vessel according to claim 2, wherein
the wedge-shaped tip is made of a polymer composite material and is integrally formed by thermal processing, and the wedge-shaped tip is omnidirectionally bendable to 360 degrees without fracture.

4. The navigation-assisted thrombectomy system for use in the cerebral vessel according to any one of claims 1 to 3, wherein
the reperfusion device consists of 10 to 60 segments and has a hardness that gradually decreases from the proximal end of the reperfusion device to the distal end of the reperfusion device, and a rate of change in hardness between two adjacent segments arranged from the distal end of the reperfusion device to the proximal end of the reperfusion device is less than 75%, preferably less than 67%.

5. The navigation-assisted thrombectomy system for use in the cerebral vessel according to any one of claims 1 to 3, wherein
a main body of the reperfusion device is a catheter, and the catheter has an outer diameter ranging from 0.8 to 0.12 inches, preferably from 0.85 to 0.115 inches.

6. The navigation-assisted thrombectomy system for use in the cerebral vessel according to claim 5, wherein
the catheter, which serves as the main body of the reperfusion device, has a length ranging from 110 to 150 cm, preferably from 120 to 145 cm.

7. The navigation-assisted thrombectomy system for use in the cerebral vessel according to claim 1, wherein
the outer radial side of the navigation device is in fit with the inner radial side of the reperfusion device, a difference between an outer diameter of the navigation device and an inner diameter of the reperfusion device is smaller than or equal to 0.005 inches, so as to cooperate with the wedge-shaped tip of the navigation device to eliminate a ledge effect.

8. The navigation-assisted thrombectomy system for use in the cerebral vessel according to any one of claims 1 to 3, wherein
a polymer composite material of the wedge-shaped tip contains contrast agent particles.

9. The navigation-assisted thrombectomy system for use in the cerebral vessel according to claim 1, further comprising a distal access guide device, wherein
the distal access guide device is configured to be advanced to a distal-most vascular position before the navigation device enters the cerebral vessel; and
the navigation device and the reperfusion device are configured to pass through the distal access guide device, pass through a vessel bend close to an ophthalmic artery, and enter the cerebral vessel.

10. The navigation-assisted thrombectomy system for use in the cerebral vessel according to claim 9, wherein
a core layer of the distal access guide device consists of 5 to 40 segments, the distal access guide device has a hardness that gradually decreases from a proximal end of the distal access guide device to a distal end of the distal access guide device, wherein a proximal-most segment of the distal access guide device has a maximum hardness, a distal-most segment of the distal access guide device has a minimum hardness, and a rate of change in hardness between two adjacent segments arranged from the distal end of the distal access guide device to the proximal end of the distal access guide device is less than 75%, preferably less than 67%.
